# EUROPÄISCHE PATENTSCHRIFT

(11) **EP 1 791 823 B1**
(45) Veröffentlichungstag und Bekanntmachung des Hinweises auf die Patenterteilung: **11.05.2011**
(21) Anmeldenummer: 05786953.9
(22) Anmeldetag: 21.09.2005
(51) Int. Cl.: C07D 313/00, A61K 31/335, A61K 31/04

(54) **ANTIBIOTIKUM UND VERFAHREN ZUR HERSTELLUNG**
ANTIBIOTIC AND METHOD FOR PRODUCTION THEREOF
ANTIBIOTIQUE ET PROCEDE DE FABRICATION

(30) Priorität: 21.09.2004 DE 102004046024
(43) Veröffentlichungstag der Anmeldung: 06.06.2007
(73) Patentinhaber: Helmholtz-Zentrum für Infektionsforschung GmbH, 38124 Braunschweig (DE)
(72) Erfinder: TIMMIS, Kenneth N., 38300 Wolfenbüttel (DE); MOLINARI, Gabriella, 38304 Wolfenbüttel (DE); JANSEN, Rolf, 38124 Braunschweig (DE); ROMERO-TABAREZ, Magally, 38124 Braunschweig (DE); SANTOSA, Dwi Andreas, 16710 Bogor (ID)
(74) Vertreter: Taruttis, Stefan Georg
(86) Internationale Anmeldenummer: PCT/EP2005/054736
(87) Internationale Veröffentlichungsnummer: WO 2006/032683

(56) Entgegenhaltungen:
- JARUCHOKTAWEECHAI, CHUTIMA ET AL: "New macrolactins from a marine Bacillus sp. Sc026" JOURNAL OF NATURAL PRODUCTS , 63(7), 984-986 CODEN: JNPRDF; ISSN: 0163-3864, 2000, XP002352574
- SMITH A B ET AL: "Total Synthesis of (-)-Macrolactin A" JOURNAL OF THE AMERICAN CHEMICAL SOCIETY, AMERICAN CHEMICAL SOCIETY, WASHINGTON, DC, US, Bd. 118, 1996, Seiten 13095-13096, XP002207324 ISSN: 0002-7863
- SMITH, A.B.; OTT, G.R.: J. AM. CHEM. SOC., Bd. 120, 1998, Seiten 3935-3948, XP002352575 in der Anmeldung erwähnt
- MARINO, J.P.; ET AL.: J. AM. CHEM .SOC., Bd. 124, Nr. 8, 2002, Seiten 1664-1668, XP002352576 in der Anmeldung erwähnt

## Beschreibung

Die vorliegende Erfindung betrifft ein neues Antibiotikum aus der Familie der Macrolactine sowie ein mikrobielles Verfahren zur Herstellung von Macrolactinen und dessen Produzentenstamm.

Es ist bekannt, dass Bakterien, insbesondere pathogene Bakterien, zunehmend resistent gegen bekannte Antibiotika werden. Ein Beispiel dafür sind Staphylokokken, eine Ursache für nosokomiale Infektionen, die auch gegen Methicillin und andere bekannte Antibiotika mit Ausnahme von Vancomycin und Teicoplanin resistent sind. Mittlerweile wird jedoch auch über das Auftauchen von Staphylokokkus-Stämmen berichtet, die gegen Vancomycin oder dessen Vorstufe resistent sind.

Es ist daher ein Ziel der vorliegenden Erfindung, neue medizinisch wirksame Substanzen bereitzustellen, insbesondere solche mit antibakterieller Wirkung, die als Antibiotikum gegenüber bakteriellen und/oder eurkaryotischen Infektionen verwendet werden können.

Es ist eine weitere Aufgabe der vorliegenden Erfindung, ein Verfahren zur Herstellung von pharmazeutisch anwendbaren Substanzen, beispielsweise solchen mit antibiotischer Wirkung bereitzustellen, sowie ein Bakterium, das als Produzent der antibiotischen Substanzen in diesem Verfahren verwendet werden kann.

### Stand der Technik

Es sind mehrere Macrolactine bekannt, darunter auch das Macrolactin A mit der chemischen Bezeichnung 8, 14, 16-Trihydroxy-24(R)-methyl-oxacyclotetracosa-3(Z), 5(E), 9(E), 11(Z), 17(E), 19(E)-hexaen-2-on. Von Macrolactin A ist bekannt, dass es antirviral, beispielsweise gegen HIV wirkt und cytotoxisch ist. Die antibakterielle Wirkung von Macrolactin A ist hingegen relativ gering.

Aus JP 9301970 ist das Derivat Macrolactin M bekannt, das antimikrobiell wirkt.

Jaruchoktaweechai et al. (J. Nat. Prod., 2000, 63, 984-986) beschreiben 7-O-Succinyl-Macrolactin F und 7-O-Succinyl-Macrolactin A, deren Isolierung aus *Bacillus* und die antibakterielle Aktivität gegen *Bacillus subtilis* und *Staphylococcus aureus.*

Chemische Syntheseverfahren für Macrolactin A sind aus Smith et al., J. Am. Chem. Soc. 120, 3935-3948 (1998) Smith et al., J. Am. Soc. 118, 13095 - 13096 (1996) und Marino et al., J. Am. Chem. Soc. 124, 8, 1664 - 1668 (2002) bekannt.

### Allgemeine Beschreibung der Erfindung

In einem ersten Aspekt stellt die Erfindung 7-O-Malonylmacrolactin A bereit, das eine gute antibakterielle Wirkung aufweist und deshalb pharmazeutischen Zusammensetzungen eine antibiotische Wirkung verleihen kann. Des Weiteren weist das 7-O-Malonylmacrolactin A insbesondere bei niedrigen Konzentrationen eine geringere zytotoxische Wirkung als Macrolactin A auf und ist auch deshalb besonders zur Verwendung in pharmazeutischen Zusammensetzungen geeignet.

Neben der allgemeinen antibakteriellen Wirkung ist 7-O-Malonylmacrolactin A auch gegen zumindest einige Bakterien antibiotisch wirksam, die Resistenzen gegenüber bekannten Antibiotika tragen oder von sich aus resistent sind. In diesem Zusammenhang hat 7-O-Malonylmacrolactin A bei Konzentrationen unterhalb der minimalen Hemmkonzentration interessanterweise eine stärkere bakteriostatische Wirkung zumindest gegenüber einigen Bakterien aus klinischen Isolaten, die Resistenzen gegen bekannte Antibiotika tragen, als gegenüber den entsprechenden nicht-resistenten Stämmen. Als Beispiele können die antibiotische Wirkung von 7-O-Malonylmacrolactin A gegenüber Methicillin-resistenten *Staphylococcus aureus* und gegenüber einem Vancomycin- und Ampicillin-resistenten *Enterococcus faecium* genannt werden.

Die Strukturformel von 7-O-Malonylmacrolactin A ist nachfolgend wiedergegeben:

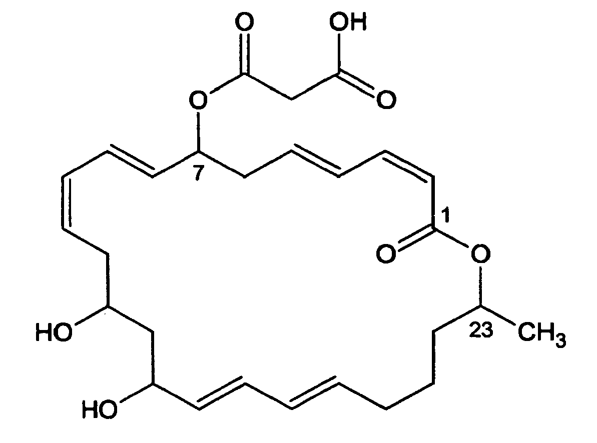

Die erfindungsgemäße, als pharmazeutische Wirkstoffe geeignete Verbindung wird von dem beim Indonesian Center for Biodiversity and Biotechnology unter der Nr. ICBB 1582 hinterlegten *Bacillus subtilis* produziert (auch hinterlegt unter Nr. DSM 16696 bei der DSMZ, Mascheroder Weg 1, 38124 Braunschweig, Hinterlegungsdatum ist der 6. September 2004). Dieser Stamm wurde aus einer Erdprobe isoliert, die aus Takalar, Südsulawesi in Indonesien stammt.

Der hinterlegte Produzentenstamm wurde als Gram-positives Stäbchen, das Endosporen (Durchmesser ±0,2 µm) bildet bestimmt, das mit Flagellen beweglich ist und auf Medium (5 g/L Hefe-Extrakt, 20 g/L Trypton, 5 g/L Natriumchlorid, 5 g/L Glucose, 15 g/L Agar) opake, milchig-weiße Kolonien mit gewellter rauher Kante bildet. In dem API-System (Biomerieux) wurden folgende positive Reaktionen festgestellt: Oxidase, Ornithin, Mannitol, Voges-Proskauer, Citrat, TDA und Stärkehydrolyse; negative Reaktionen wurden festgestellt für Nitrat, Lysin, Produktion von Schwefelwasserstoff, Glucose, Xylose, ONPG, Indol und Urease. Nach den biochemischen Tests und der Homologie-Suche der Sequenz der 16S-RNA mit FASTA wurde der Stamm als *Bacillus subtilis* identifiziert.

In einem weiteren Aspekt betrifft die vorliegende Erfindung fermentative Herstellungsverfahren sowohl für das bekannte Macrolactin A, 7-O-Succinylmacrolactin A, als auch das 7-O-Malonylmacrolactin A.

Weiterhin betrifft die vorliegende Erfindung die Verwendung von 7-O-Malonylmacrolactin A zur Herstellung von pharmazeutischen Präparaten zur medizinischen Anwendung gegen antibiotika-resistente, z.B. multiresistente Bakterien.

Daher stellt die vorliegende Erfindung auch pharmazeutische Zusamnnensetzungen bereit, die einen Gehalt der vorgenannten Verbindung aufweist.

### Genaue Beschreibung der Erfindung

### 7-O-Malonylmacrolactin A

7-O-Malonyl-Macrolactin A ist gegenüber Bakterien, insbesondere gegenüber Gram-positiven Bakterien antibiotisch wirksam und, besonders vorteilhaft, bei sehr niedrigen Konzentrationen, beispielsweise im Bereich von 0,05-4 µg/mL, bakteriostatisch. Dieser Effekt ist insbesondere gegenüber antibiotikaresistenten Bakterien zu beobachten, so dass 7-O-Malonylmacrolactin A besonders geeignet ist, gegenüber antibiotikaresistenten Bakterien, darunter insbesondere Gram-positive Bakterien, zumindest deren weitere Vermehrung effektiv zu hemmen. Diese bakteriostatische Wirkung von 7-O-Malonylmacrolactin A tritt bereits bei niedrigeren Konzentrationen auf, als z.B. bei dem bekannten Macrolactin A, selbst wenn eine direkte und unmittelbare bakterizide Wirkung bei diesen niedrigen Konzentrationen nicht eintritt.

Als weiteren Vorteil gegenüber dem bekannten 7-O-Succinylmacrolactin A oder Macrolactin A hat 7-O-Malonylmacrolactin A selbst gegenüber antibiotikaresistenten Bakterien schon bei sehr niedrigen Konzentrationen eine bakteriostatische Wirkung. Solche antibiotikaresistenten Bakterien können z.B. aus klinischen Isolaten stammen und gegenüber Erythromycin oder Vancomycin resistent sein, oder mehrfache Resistenzen tragen, darunter z.B. *Staphylococcus aureus* (MRSA) oder Enterokokken (VRE) aus klinischen Proben. Neben der antibakteriellen Wirkung gegen diese Gram-positiven Bakterien wirkt 7-O-Malonylmacrolactin A auch gegen Gram-negative Bakterien, z.B. die kleine Kolonien bildende Variante (SCV) von *Burkholderia cepacia.* Dabei ist die antibakterielle Wirkung gegenüber antibiotikaresistenten Stämmen und der SCV ausgeprägter als gegenüber Wildtypstämmen. Bei höheren Konzentrationen hemmt 7-O-Malonylmacrolactin A auch eukaryontische Mikroorganismen, z.B. pathogene Hefen, insbesondere *Candida krusei.*

Entsprechend der bakteriostatischen Wirkung wird 7-O-Malonylmacrolactin A bevorzugt zur Herstellung pharmazeutischer Präparate für die folgenden medizinischen Indikationen eingesetzt: bakterielle Infektionen, Endokarditis, Meningitis, Osteomyelitis, darunter auch Infektionen durch toxinbildende Erreger, z.B. Streptokokken und Staphylokokken, insbesondere toxisches-Schocksyndrom (TSS)-Stämmen von *Staphylococcus aureus.*

Weitere medizinische Indikationen, gegen die die erfindungsgemäßen Zusammensetzungen verwendet werden können, sind Atemwegsinfekte, insbesondere durch antibiotika-resistente (z.B. gegen Penicillin resistente) Bakterien, oder durch Bakterien, die für herkömmliche Antibiotika schwer erreichbar sind, wie z.B. oberflächlich angesiedelte Erreger. Beispiele für Atemwegsinfekte sind Infektionen durch extrazelluläre und/oder intrazelluläre Bakterien, wie beispielsweise Streptokokken, z.B. *Streptococcus pneumoniae* oder *Streptococcus pyogenes.*

Für die medizinische Anwendung ist die Eigenschaft von 7-O-Malonylmacrolactin A von Bedeutung, bei niedrigen Konzentrationen, die deutlich unterhalb der MHC liegen können, bakteriostatisch zu wirken, dies bevorzugt gegenüber klinisch relevanten Stämmen, z.B. mit Resistenzen. Bereits bei niedrigen Konzentrationen beeinträchtigt 7-O-Malonylmacrolactin sowohl bei Gram-positiven als auch Gram-negativen Bakterien A die Zellteilung, was sich elektronenmikroskopisch anhand einer gestörten Septenbildung und Zellwandveränderungen beobachten lässt. So zeigen z.B. anitbiotika-resistente Enterokokken, insbesondere VRE, und Staphylokokken, insbesondere MRSA, und *Burkholderia cepacia* SCV in Gegenwart von 7-O-Malonylmacrolactin A eine verdickte Zellwand und/oder eine anomale Morphologie. Die Störung der Zellteilung führt zu pseudo-multizellulären Anhäufungen, die nicht in der Lage sind, separate Tochterzellen zu bilden. Derzeit wird angenommen, dass die Verminderung der Virulenz bakterieller Infektionen durch 7-O-Malonylmacrolactin A auch auf die Störung der Zellteilung zurückzuführen ist, da auf diese Weise die Ausbreitung der Bakterien verzögert bzw. verhindert wird.

7-O-Malonylmacrolactin A wird von dem *Bacillus subtilis* Stamm mit der Hinterlegungs-Nr. DSM 16696 produziert und ins Kulturmedium ausgeschieden. Aus dem Kulturmedium lässt sich 7-O-Malonylmacrolactin A ebenso wie 7-O-Succinylmacrolactin A und Macrolactin A gewinnen, beispielsweise durch Adsorption an ein hydrophobes Adsorber-Harz wie XAD, nachfolgendes Waschen des Harzes mit wässrigem Methanol und Elution mit 100% Methanol. Aus diesem Eluat läßt sich 7-O-Malonylmacrolactin A isolieren.

7-0-Malonylmacrolactin A: C₂₇H₃₆O₈, M = 488,57. UV (MeOH) λₘₐₓ (lg ε) = 227 nm (4,397), 230 (sh), 260 (4,006). [α]²²_{D} = -6,2 (c = 0,63 in MeOH), MS: (-)-ESI (TOF): m/z (%) = 487,2 (100) [M-H]⁻, 443,2 (44) [M-H-CO₂]⁻, 383,2 (27) [M-H-Malonsäure]⁻.

Diese Verbindung wurde aufgrund ihres mit demjenigen von Macrolactin A identischen UV-Spektrum als Verbindung vom Macrolactin-Typ identifiziert. Die Massenspektrometrie zeigte eine Molekülmasse von 488, was um 86 höher liegt als die Masse, die für Macrolactin A beobachtet wurde. Entsprechend der Eliminierung eines H₂O aus Macrolactin A zeigte 7-O-Malonylmacrolactin A die Abspaltung von Malonsäure durch einen Ionen-Fragment bei m/z 383 in dem (-)-ESI-Spektrum. Die NMR-Daten von 7-O-Malonylmacrolactin A sind in Tabelle 1 angegeben. Diese waren im Wesentlichen identisch zu den NMR-Daten von Macrolactin A, wobei jedoch das Signal von 7-H als eine Folge der Acylierung des 7 - O um etwa 1,2 ppm tieffeldverschoben war. Der an das 7-O gebundene Rest wurde aus NMR-Spektren im Vergleich mit Macrolactin A identifiziert. Die nur eine Carboxygruppe wurde direkt aus den NMR-Spektren in Dichlormethan-d₄ hergeleitet, die das Vorliegen eines Malonylrestes durch zusätzliche Carboxy-¹³C-Signale bei 166,33 und 169,15 ppm und ein Methylen-¹³C-Signal bei 42,17 ppm anzeigten. Die zugehörigen Methylen-¹H-Signale wurden als Dubletts bei 3,51 und 3,40 ppm mit *J*= 15,5 Hz beobachtet In Methanol-d₄ war nur ein Carboxy-¹³C-Signal sichtbar.

**Tabelle 1: NMR-Daten von Macrolactin A und 7-O-Malonylmacrolactin A in Methanol-d₄**

| Macrolactin A | | | | | | | 7-*O*-Malonylmacrolactin A | | | | | | |
|---|---|---|---|---|---|---|---|---|---|---|---|---|---|
| **H** | **δ_{H}** | **m** | ***J* [Hz]** | **C** | δ**_{C}** | **m** | **H** | **δ_{H}** | **m** | ***J* [Hz]** | **C** | **δ_{C}** | **m** |
| **1** | - | - | - | **1** | 168.02 | s | **1** | - | - | - | **1** | 167.94 | s |
| **2** | 5.58 | d | 11.33 | **2** | 118.00 | d | **2** | 5.59 | d | 11.7 | **2** | 118.52 | d |
| **3** | 6.67 | t | 11.71 | **3** | 144.96 | d | **3** | 6.67 | t | 11.3 | **3** | 144.50 | d |
| **4** | 7.26 | dddd | 15.1,11.4,2,1 | **4** | 130.26 | d | **4** | 7.25 | dd | 14.7, 11.7 | **4** | 130.79 | d |
| **5** | 6.20 | m | - | **5** | 142.16 | d | **5** | 6.15 | dt | 15.4, 7.2 | **5** | 140.51 | d |
| **6** | 2.45 | m | - | **6** | 42.84 | t | **6** | 2.60 | m | 5.3 | **6** | 40.13 | t |
| **7** | 4.29 | ddt | 4.9,1.2, 6.8 | **7** | 72.33 | d | **7** | 5.50 | ddd | 6.0, 6.0, 6.0 | **7** | 74.72 | d |
| **8** | 5.79 | dd | 15.1,6.0 | **8** | 137.55 | d | **8** | 5.75 | dd | 15.3,5.5 | **8** | 132.06 | d |
| **9** | 6.61 | ddt | 15.2, 11.0,1.1 | **9** | 125.96 | d | **9** | 6.71 | dd | 15.1,11.3 | **9** | 128.09 | d |
| **10** | 6.15 | t | 11.14 | **10** | 131.39 | d | **10** | 6.13 | t | 10.2 | **10** | 130.91 | d |
| **11** | 5.58 | ddd | 10.5, 8.6, 8.2 | **11** | 128.39 | d | **11** | 5.63 | dt | 10.6, 8.4 | **11** | 129.78 | d |
| **12a** | 2.53 | dddd | 13.5,8.2,7.4,0.8 | **12** | 36.50 | t | **12a** | 2.63 | m | | **12** | 36.39 | t |
| **12b** | 2.36 | dddd | 13.5, 7.7, 4.9, 1.1 | | | | **12b** | 2.33 | ddd | 13.0, 7.2, 5.5 | | | |
| **13** | 3.89 | ddt | 5.3, 5.1, 6.9 | **13** | 69.24 | d | **13** | 3.84 | ddd | 10.6,6.0,5.7 | **13** | 69.51 | d |
| **14** | 1.65 | m | - | **14** | 43.92 | t | **14** | 1.66 | m | | **14** | 43.84 | t |
| **15** | 4.34 | dt | 6.3, 6.3 | **15** | 69.83 | d | **15** | 4.39 | dt | 6.3, 6.3 | **15** | 69.77 | d |
| **16** | 5.60 | dd | 15.1,6.5 | **16** | 135.23 | d | **16** | 5.60 | dd | 15.1,6.4 | **16** | 135.32 | d |
| **17** | 6.21 | dd | 15.5, 10.9 | **17** | 131.21 | d | **17** | 6.21 | dd | 15.1, 10.6 | **17** | 131.27 | d |
| **18** | 6.09 | dd | 14.9, 10.4 | **18** | 131.72 | d | **18** | 6.10 | dd | 15.1, 10.6 | **18** | 131.78 | d |
| **19** | 5.69 | ddd | 14.7,7.2,6.8 | **19** | 135.13 | d | **19** | 5.69 | ddd | 14.9, 7.0, 6.8 | **19** | 135.10 | d |
| **20a** | 2.23 | ddt | 142, 7.1, 6.8 | **20** | 32.98 | t | **20a** | 2.23 | td | 14.0, 6.8 | **20** | 33.03 | t |
| **20b** | 2.14 | ddt | 14.2, 6.4, 7.1 | | | | **20b** | 2.15 | td | 14.4, 7.2 | | | |
| **21** | 1.54 | m | - | **21** | 25.65 | t | **21** | 1.54 | m | - | **21** | 25.81 | t |
| **22a** | 1.68 | m | - | **22** | 36.01 | t | **22a** | 1.70 | m | - | **22** | 36.08 | t |
| **22b** | 1.61 | m | - | | | | **22b** | 1.62 | m | - | | | |
| **23** | 5.05 | ddq | 7.3, 4.5, 6.2 | **23** | 72.21 | d | **23** | 5.05 | ddq | 4.5,7.1,6.1 | **23** | 72.37 | d |
| **24** | 1.29 | d | 6.04 | **24** | 20.11 | q | **24** | 1.30 | d | 6.0 | **24** | 20.14 | q |
| **1'** | - | - | - | - | - | - | **1'** | - | - | - | **1'** | 169.64 | s |
| **2'** | - | - | - | - | - | - | **2'** | 2.90 | m | - (br) | **2'** | 44.74 | (a) |
| **3'** | - | - | - | - | - | - | **3'** | - | - | - | **3'** | n.b. | |

| | | | | | | | | | | | | | |
|---|---|---|---|---|---|---|---|---|---|---|---|---|---|
| ¹H bei 600 MHz; ¹³C at 150 MHz; (a) aus HMQC-NMR Spectrum. n.b.= nicht beobachtet aufgrund Signalverbreiterung. Die Multiplizität der Kohlenstoffsignale wurde aus DEPT- und HMQC-Spektren erhalten. | | | | | | | | | | | | | |

### Macrolactin A

Macrolactin A: C₂₄H₃₅O₅, M = 402,53. UV (Methanol): λₘₐₓ(lg ε) = 227 nm (4,537), 261 (4,146) [Lit.: 227 (4,691), 261 (4,272)]. [α]²²D =-10,7 (c = 0,68 in MeOH) [Lit.: -9,6 [c = 1,86 in MeOH)]. MS: (-)-ESI (TOF): *m*/*z* (%) = 401,2 (38) [M-H]⁻, 437,2 (100) [M+Cl]⁻, 803,4 (63) [2M-H]⁻; (-)-DCI (Isobutan): *m*/*z* (%) = 402 (100); (+)-DCI (Isobutan): *m*/*z* (%) = 349 (56) [M+H - 3H₂O]⁺, 367 (100); [M+H -2H₂O]⁺, 385 (75) [M+H -H₂O]⁺; EI (200 °C): *mlz* (%) = 255 (100), 273 (72), 348 (18), 366 (68), 384 (60), 400 (5,9), 402 (2,5). Die NMR-Daten sind oben in Tabelle 1 angegeben.

### 7-O-Succinylmacrolactin A

C₂₈H₃₈O₈, M = 502,60: UV (MeOH) λₘₐₓ (lg ε) = 227 nm (4,596), 259 (4,192) [Lit.: 229 (4,57), 261 (4,18)]. [α]²²_{D} = -19,9 (c = 0,7 in MeOH) [Lit.: - 9,6 (c = 0,18 in MeOH). MS: (-)-ESI (TOF): m/z (%) = 501,3 (100) [M-H]⁻, 117,0 (12) [Bernsteinsäure-H]⁻; (-)-DCl (Isobutan): m/z (%) = 502,7 (100), 484 (44), 402 (18), 384 (68), 366 (26), 117 (20).

Die ¹H-NMR-Daten in CDCL₃ waren identisch mit denjenigen, die von Jaruchktaweechai et al., J. Nat. Prod. 63, (7), 984-986 (2000) bestimmt wurden.

Die chemischen Eigenschaften der drei vorgenannten Macrolactin-Verbindungen, die von *Bacillus subtilis* DSM 16696 produziert werden, sind in der nachfolgenden Tabelle 2 zusammengefaßt:

**Tabelle 2:**

| Eigenschaft | Macrolactin A | 7-*O*-Malonyl-macrolactin A | 7-*O*-succinyl-macrolactin A |
|---|---|---|---|
| Summenformel | C₂₄H₃₅O₅ | C₂₇H₃₆O₈ | C₂₈H₃₈O₈ |
| Molekulargewicht | 402,53 | 488,57 | 502,60 |
| UV (MeOH) [λₘₐₓ (lg ε)] | 227 (4,537) 261(4,146) | 227 (4,397), 230 (sh) 260 (4,006) | 227 (4,596) 259 (4,192) |
| [α]²²D (*c* in MeOH) | - 10,7 (0,68) | - 6,2 (0,63) | - 19,9 (0,7) |
| (-)-MS-ESI-TOF [*m*/*z* (%)] | 401,2 (38) [M-H]⁻ 437,2 (100) [M+Cl]⁻ 803,4 (63) [2M-H]⁻ | 487,2 (100) [M-H]⁻ 443,2 (44) [M-H-CO₂]⁻ 383,2 (27) [M-H-Malonsäure]⁻ | 501,3 (100) [M-H]⁻ 117,0 (12) [Bernsteinsäure-H]⁻ |

### Medizinische Verwendung von 7-O-Succinylmacrolactin A gegen antibiotika-resistente Bakterien

Entsprechend dem oben beschriebenen 7-O-Malonylmacrolactin A zeigt auch 7-O-Succinylmacrolactin A eine stärkere Wirkung gegen antibiotika-resistente Bakterien, wie z.B. multiresistente Bakterien, insbesondere gegen Bakterien, die beispielsweise gegen eines von Vancomycin, Erythromycin, Methicillin bzw. Ampicillin resistent sind.

Daher stellt die vorliegende Erfindung auch die Verwendung von 7-O-Succinylmacrolactin A zur Herstellung pharmazeutischer Präparate bereit, die gegen antibiotika-resistente Bakterien wirksam sind. Entsprechend der besonderen bakteriostatischen Wirkung gegen antibiotika-resistente Bakterien bei niedrigen Konzentrationen unterhalb der MHC, d.h. im Bereich der MBC von 7-O-Succinylmacrolactin A und 7-O-Malonylmacrolactin A können solche pharmazeutischen Präparate 7-O-Succinylmacrolactin A und 7-O-Malonylmacrolactin A einzeln oder in Kombination miteinander enthalten. Die Kombination von 7-O-Succinylmacrolactin A und 7-O-Malonylmacrolactin A ist besonders bevorzugt, da die bakteriostatische Wirkung zumindest gegenüber einigen antibiotika-resistenten Bakterien unterschiedlich effektiv ausfällt.

### Fermentative Herstellungsverfahren

*B. subtilis* DSM 16696 kann zur Herstellung von 7-O-Malonylmacrolactin A, 7-O-Succinylmacrolactin A und/oder Macrolactin A verwendet werden.

In den nachfolgenden Beispielen ist die fermentative Herstellung mittels Schüttelkultur beschrieben worden, es lassen sich jedoch auch bekannte Submersverfahren in Fermentern einsetzen, um größere Volumina zu kultivieren. Dies schließt Batch-, Fed-Batch- und kontinuierliche Fermentationsverfahren ein.

Die Erfindung wird nun detailliert anhand von Beispielen beschrieben. Die in Bezug genommenen Figuren zeigen in
- Figur 1A die Wachstumskinetik für *Staphylococcus aureus* (#) ohne (0) und mit 1 µg/mL (1) bzw. 4 µg/mL (4) 7-O-Malonylmacrolactin A,
- Figur 1B die Wachstumskinetik für *Staphylococcus aureus* (Methicillin resistent aus klinischem Isolat, MRSA 3) ohne (0) und mit 1 µg/mL (1) bzw. 4 µg/mL (4) 7-O-Malonylmacrolactin A,
- Figur 1C die Wachstumskinetik für *Enterococcus faecalis* ATCC 29212 ohne (0) und mit 4 µg/mL (4) bzw. 16 µg/mL (16) 7-O-Malonylmacrolactin A,
- Figur 1D die Wachstumskinetik für *Enterococcus faecium* (VRAR E315, Vancomycin resistent, Ampicillin resistent, klinisches Isolat) ohne (0) und mit 4 µg/mL (4) bzw. 16 µg/mL (16) 7-O-Malonylmacrolactin A,
- Figur 1E die Wachstumskinetik für *Candida krusei* DSMZ6128 ohne (0) und mit 32 µg/mL (32) bzw. 128 µg/mL (128) 7-O-Malonylmacrolactin A
- Figur 1F die Wachstumskinetik für *Burkholderia cepacia* SCV 141 (klinisches Isolat) ohne (0) und mit 32 µg/mL (32) bzw. 128 µg/mL (128) 7-O-Malonylmacrolactin A,
- Figur 2 bei a), b) und c) elektronenmikroskopische Aufnahmen des *S. aureus* MRSA 3 nach 4 Stunden Wachstum ohne Antibiotikum und bei d), e) und f) in Gegenwart von 16 µg/mL 7-O-Malonylmacrolactin A,
- Figur 3 bei a) bis c) elektronenmikroskopische Aufnahmen des *E. faecium* VRAR E315 nach 4 Stunden Wachstum ohne Antibiotikum und bei d), e) und f) in Gegenwart von 16 µg/mL 7-O-Malonylmacrolactin A,
- Figur 4 bei a) bis c) elektronenmikroskopische Aufnahmen von *Burkholderia cepacia* SCV nach 4 Stunden Wachstum ohne Antibiotikum und bei d), e) und f) in Gegenwart von 128 µg/mL 7-O-Malonylmacrolactin A,
- Figur 5A die Hemmung der Proliferation von Mauszellen L-929 durch 7-O-Malonylmacrolactin A (•), 7-O-Succinylmacrolactin A (▼) und Macrolactin A (■), und
- Figur 5B die Hemmung der Proliferation von menschlichen Epithelzellen (HeLa, ▼) und Mauszellen L-929 (•) durch 7-O-Malonylmacrolactin A.

### Beispiel 1: Kultivierung von Bacillus subtilis zur Produktion von 7-O-Malonylmacrolactin A

Der Produzentenstamm DSM 16696 wurde in Medium mit 5 g/L Hefe-Extrakt, 20 g/L Trypton, 5 g/L Natriumchlorid und 5 g/L Glucose bei pH = 7 in Flüssigkultur angezogen. Während der Kultivierung in Schüttelkultur (120 Upm) über 7 Tage bei 30 °C lagen 4 Gew.-% des hydrophoben Adsorberharzes Amberlite XAD-16 (Röhm und Haas, Deutschland) in dem Kulturmedium vor.

Das durch Dekantieren abgetrennte Adsorberharz wurde in einer Säule mit 50-prozentigem wässrigem Methanol gewaschen, adsorbierte Verbindungen wurden mit Methanol eluiert und durch Einrotieren auf 1/100 des Volumens der Kultur aufkonzentriert. Aus dem Extrakt wurde das Methanol durch Verdampfen entfernt, die verbleibende wässrige Mischung wurde 4-mal mit Ethylacetat extrahiert. Unter vermindertem Druck wurde das Ethylacetat entfernt, es wurden ca. 300 mg öligen Rückstands von insgesamt 4 L Kultur, der Adsorberharz zugegeben war, erhalten. Dieser Rückstand wurde in Methanol aufgenommen und 4-mal im gleichen Volumen n-Heptan ausgeschüttelt, um die lipophileren Bestandteile und Verunreinigungen zu entfernen.

### Beispiel 2: Kultivierung von Bacillus subtilis zur Produktion von 7-O-Malonylmacrolactin A

Zur Produktion wurde entsprechend Beispiel 1 der Produzentenstamm angezogen, wobei jedoch das Medium OM bei pH = 7 verwendet wurde. Dieses Medium ist zur Produktion von Macrolactinen vorteilhaft, da die nachfolgende Isolierung bei gleichen Schritten zu höherer Reinheit führt.

OM Medium wird durch Autoklavieren von 1,0 g Stärke, 1,0 g Glucose, 1,0 g Pepton und 1,5 g Hefeextrakt pro 980 mL Wasser und Zugeben von 10 mL/L Lösung A (5g/L KH₂PO₄, 5 g/L K₂HPO₄, autoklaviert), 10 mL/L Lösung B (17 g/L MgSO₄, 1,0 g/L NaCl, 0,7 g/L MnSO₄, 0,06 g/L CuSO₄, autoklaviert), 1 mL/L Lösung C (7 g/L FeSO₄-7H₂O, 22 g/L Na₃-citrat·3 H₂O, 20 g/L Ammoniumcitrat, 7,5 g/L Na-thioglycalase, 33 g/L Na2-succinat-6 H₂O, sterilfiltriert) und 1 mL/L Lösung D (100 mg/L Biotin, 350 mg/L Nicotinsäureamid, 300 mg/L Thiamin·HCl, 200 mg/L p-Aminobenzoesäure, 100 mg/L Pyridoxalhydrochlorid, 100 mg/L Ca-pantothenat, 50 mg/L Vitamin B₁₂, sterilfiltriert) nach dem Autoklavieren erhalten.

### Beispiel 3: Aufreinigung von Macrolactinen durch Flüssigkeitschromatographie

Extrakte und Fraktionen von Beispiel 1 oder 2 wurden durch Umkehrphasen-Chromatographie (RP-HPLC) mit einer Nukleosil 100-5 C18-Säule (125/2 mm, Macherey Nagel) analysiert, zur Detektion wurde ein 320-600 nm UV-Diodenarray-Detektor und ein externer Streulicht-Detektor (PL·ELS - 1000, Polymer Laboratories) eingesetzt. Als Lösungsmittel wurden A: 0,5% Essigsäure / Wasser und B: 0,5% Essigsäure / Methanol bei einer Strömungsrate von 0,3 mL/Minute verwendet. Die Macrolactine wurde durch präparative RP-HPLC unter Verwendung einer Nucleosil 100-7 C18-Säule (250/21 mm, Macherey Nagel) mit einem Gradienten von 51% bis 56% Lösungsmittel A in Lösungsmittel B bei einer Strömungsrate von 30 mL/Minute aufgetrennt. Es wurden 40 - 60 mg Extrakt in 0,2 mL Methanol aufgetragen, die UV-Detektion erfolgte bei 280 nm.

Zur Aufreinigung von Macrolactinen wurde auch die präparative RP-HPLC unter Verwendung einer Nukleosil 100-7 C18 Säule (250/21 mm, Macherey-Nagel, Düren, Deutschland), mit einem Lösungsmittelgradienten eingesetzt: Lösungsmittel A (0,5% Essigsäure / 51% wässriges Methanol) und Lösungsmittel B (0,5% Essigsäure / 56% wässriges Methanol), Gradient: Lösungsmittel B von 0 bis 100% über 60 min bei einer Strömungsrate von 30 mL/min, UV-Detektion bei 280 nm. Zur Injektion wurden 40 bis 60 mg Extrakt in 0,2 mL Methanol verwendet. Jedes Macrolactin (5-7 mg) wurde weiter durch Chromatographie an einer LH-20 Säule (760/25 mm, Lösungsmittel Methanol / Dichlormethan (1:1), Strömungsrate 5 mL/min) bei einer Auftragmenge von 5 bis 7 mg aufgereinigt.

Mit präparativer RP-HPLC und anschließender Aufreinigung durch LH-20 Chromatographie wurden aus einer Kultur von 4 L 4 - 6 mg Macrolactin A, 5 bis 7 mg 7-O-Malonylmacrolactin A und 6 bis 8 mg 7-O-Succinylmacrolactin A isoliert.

Die von dem erfindungsgemäßen *Bacillus subtilis* isolierten Macrolactin-Verbindungen führten zu den voranstehend angegebenen Analyseergebnissen.

Macrolactin A: Das Molekülion m/z = 402, UV-Absorption bei 227 und 261 nm führte zur Identifizierung als Macrolactin A oder seines 10E - Isomers, Macrolactin I. Letzteres wurde durch die optische Drehung von [α²² _{D}] = -138 im Vergleich mit -10 für Macrolactin A ausgeschlossen. Die ¹H- und ¹³C - NMR Daten für die gut aufgelösten Spektren von Macrolactin A in Methanol-d₄ sind in Tabelle 2 angegeben. Die Signale wurden durch ¹H, ¹H-COSY und ¹H-, ¹³C-HMBQ-Spektren identifiziert.

### Beispiel 4: Antimikrobielle Wirkung

Die Wirkung gegen Bakterien und Hefe wurden nach der Agar-Diffusionsmethode auf Mueller-Hinton (MH) Agar (Difco Laboratories) bestimmt. Sterile Papierscheiben

(Schleicher&Schüll, Deutschland) wurden mit 10 µL Rohextrakt oder einer Lösung aufgereinigter Macrolactine (Endkonzentration von 50 µg der Verbindung pro Scheibe) imprägniert und auf die Oberfläche von MH-Agarplatten gelegt. Die Platten waren zuvor mit einer Suspension von 10⁵ Zellen pro mL der zu testenden Mikroorganismen inokuliert worden, die aus einer Übernachtkultur stammten. Der Durchmesser der Hemmzonen um die Scheiben wurden nach 18 Stunden Inkubieren bei 37 °C gemessen.

Der Rohextrakt von Beispiel 1 zeigte eine vollständige bakterizide Wirkung mit komplett klarem Hof um die Scheibe. Dies beruht vermutlich auf der additiven Wirkung der enthaltenen Macrolactine.

Das 7-O-Malonylmacrolactin A zeigte antibakterielle Wirkung sowohl gegen Gram-positive als auch am Beispiel von *Burkholderia cepacia,* Gram-negative Referenz- und klinische Isolate mit einer Wirkung, die vergleichbar ist mit der von Erythromycin oder besser. Insbesondere gegenüber Methicillin- bzw. Ampicillin-, Vancomycin und/oder Erythromycinresistenten bakteriellen Isolaten werden die neuartigen Eigenschaften von 7-O-Malonylmacrolactin A und 7-O-Succinylmacrolactin A darin deutlich, dass diese Verbindungen auch gegen antibiotika-resistente Isolate eine antibakterielle Wirkung haben. Die Ergebnisse sind in der nachfolgenden Tabelle 3 dargestellt.

**Tabelle 3: Vergleich der antibakteriellen Wirkung in vitro von Macrolactinen und herkömmlichen Antibiotika**

| | Hemmzone (mm) | | | | | | |
|---|---|---|---|---|---|---|---|
| Teststamm | Roh-extrakt | Macrolactin A | 7-O-Malonyl-macrolactin A | 7-O-Succinyl-macrolactin A | Erythromycin | Vancomycin | Ampicillin |
| *S. aureus* DSMZ 1104** | 28 | 18 | 26 | 18 | 30 | 20 | 30 |
| *S. aureus* **,^{#} | 32 | 25 | 30 | 22 | 40 | 27 | 41 |
| MRSA 2 | 24 | 27 | 40 | 37 | 0 | 24 | 0 |
| MRSA 3 | 28 | 35 | 41 | 38 | 0 | 24 | 0 |
| *E. faecalis* ATCC 29212 | 18 | 0 | 25 | 12 | 25 | 24 | 32 |
| Teststamm | Roh-extrakt | Macrolactin A | 7-O-Malonyl-macrolactin A | 7-O-Succinyl-macrolactin A | Erythromycin | Vancomycin | Ampicillin |
| *E. faecalis* VRAS E305 | 15 | 0 | 15 | 0 | 0 | 0 | 35 |
| *E. faecium* VRAR E315 | 40 | 0 | 15 | 20 | 25 | 0 | 0 |
| *B. cepacia* SCV* 141 | 40 | 0 | 15 | 0 | 0 | Nicht bestim -mt | 0 |

| | | | | | | | |
|---|---|---|---|---|---|---|---|
| - *SCV = Kleinkolonien bildende Variante (small colony variant). - MRSA = Methicillin resistenter *S. aureus,* - VRAS = Vancomycin resistent, Ampicillin empfindlich - VRAR = Vancomycin resistent, Ampicillin resistent - ** = Kontrollstämme, Methicillin empfindlich - # = eigene Sammlung - Rohextrakt wurde zu 10 µL/Scheibe und Macrolactine wurden zu 50 µg/Scheibe aufgetragen. Erythromycin war zu 78 µg /Scheibe, Vancomycin zu 30 µg/Scheibe und Ampicillin zu 10 µg /Scheibe enthalten (vorbereitete Scheiben). | | | | | | | |

Die Ergebnisse von Tabelle 3 zeigen am Beispiel der gegen Erythromycin resistenten Stämme *E. faecalis* VRAS E305 und *B. cepacia* SCV* 141, dass das 7-O-Succinylmacrolactin A hier keine Wirkung zeigt, sondern nur 7-O-Malonylmacrolactin A eine Hemmzone auf Grund der antibakteriellen Wirkung erzeugte.

Hingegen zeigen sowohl 7-O-Succinylmacrolactin A als auch 7-O-Malonylmacrolactin A eine antibakterielle Wirkung gegen die multiresistenten Enterokokken, beispielhaft an den Staphylokokken MRSA 2 und MRSA dargestellt. Gegenüber dem ebenfalls wirksamen Macrolactin A haben 7-O-Succinylmacrolactin A und 7-O-Malonylmacrolactin A bei der erfindungsgemäßen Verwendung jedoch den Vorteil einer niedrigeren Zytotoxizität, die sie für die Verwendung als antibakterielle Wirkstoffe, insbesondere als bakeriostatische Wirkstoffe im niedrigen Konzentrationsbereich der MBC geeignet macht.

Die Ergebnisse von Tabelle 3 zeigen auch, dass 7-O-Malonylmacrolactin A eine deutlich stärkere antibakterielle Wirkung gegen alle getesteten Bakterien hat, als Macrolactin A. Einzige Ausnahme ist *E. faecium* VRAR E315, gegenüber welchem die antibakterielle Wirkung von 7-O-Malonylmacrolactin A geringfügig schwächer als die von 7-O-Succinylmacrolactin A ist. Daher ist es in einer Ausführungsform der Erfindung bevorzugt, dass die pharmazeutische Zusammensetzung mit antibakterieller Wirkung 7-O-Malonylmacrolactin A enthält, optional in Kombination mit 7-O-Succinylmacrolactin A.

Insbesondere die neue Verbindung 7-O-Malonylmacrolactin A war in der Lage, das Wachstum von Methicillin-resistenten *Staphylococcus aureus* (MRSA 2 bzw. 3, klinisches Isolat) und Vancomycin-resistentem *Enterococcus* (VRE) zu hemmen. 7-O-Malonylmacrolactin A war die aktivste der drei Macrolactin-Verbindungen, die von dem *Bacillus subtilis* produziert wurden. In der Agardiffusionsmethode wurde ein großer Hemmhof des bakteriellen Wachstums um die Scheibe beobachtet. Die Hemmung des bakteriellen Wachstums von *Staphylococcus* war jedoch nicht vollständig und es konnten kleine Kolonien innerhalb dieser Zone nachgewiesen werden.

Der verwendete Stamm von *B. cepacia* ist ein klinisches Isolat aus einem Patienten mit zystischer Fibrose und bildet Kleinkolonien (SCV 141). Dieses Isolat weist daher die typischen Eigenschaften auf, die Bakterien in Kombination mit mukoiden Bakterien in einem Biofilm aufweisen können, der aufgrund der höheren Antibiotika-Resistenz große klinische Bedeutung hat und sich insbesondere bei Patienten mit zystischer Fibrose bildet. In diesem Vergleichstest zeigte allein 7-O-Malonylmacrolactin A eine antibakterielle Wirkung gegenüber dem Isolat von *B. cepacia* mit Eigenschaften, die die Eignung für die medizinische Anwendung zeigen.

### Beispiel 5: minimale Hemmkonzentrationen von 7-O-Malonylmacrolactin A

Die minimalen Hemmkonzentrationen (MHC) von 7-O-Malonylmacrolactin A lagen für Staphylokokken oberhalb von 128 µg/mL, jedoch wurde zwischen 1 und 4 µg/mL eine starke Hemmung des Bakterienwachstums für den Referenzstamm *(S. aureus* ^{#}*,* nicht Methicillinresistent) bzw. für die MRSA-Stämme nachgewiesen. Der MHC-Wert von 7-O-Malonylmacrolactin A gegen *Enterococcus faecalis* ATCC 29212 lag oberhalb von 128 µg/mL und der sub-MHC-Wert, bei dem das bakterielle Wachstum stark inhibiert wurde, betrug 4 µg/mL. 7-O-Malonylmacrolactin A zeigte MHC-Werte oberhalb 128 µg/mL gegen VRE-Stämme, hemmte das bakterielle Wachstum jedoch deutlich bei 0,06 bzw. 4 µg/mL für die Stämme *Enterococcus faecalis* E 305, Vancomycin-resistent / Ampicillin-empfindlich (VRAS) bzw. *Enterococcus faecium* E 315, Vancomycin- resistent / Ampicillin-resistent (VRAR).

7-O-Malonylmacrolactin A war gegen einige der von den Erfindern getesteten Gram-negativen Bakterien nicht wirksam. Es wurde jedoch eine Hemmung des Wachstums klinischer Isolate Gram-negativer Bakterien beobachtet, z.B. von *Burkholderia cepacia* SCV 141 bei Konzentrationen von 32 µg/mL, jedoch nicht gegenüber dem Wildtyp-Stamm *Burkholderia cepacia* 139, für den der MHC-Wert mehr als 128 µg/mL betrug. Dies zeigt jedoch, dass auch gegenüber klinisch bedeutsamen Gram-negativen Stämmen, insbesondere solchen mit zumindest einer Resistenz gegen ein Antibiotikum, 7-O-Malonylmacrolactin A für die medizinische Verwendung geeignet ist.

Weiterhin wurde auch eine Hemmwirkung gegenüber *Candida* spp. festgestellt.

Die starke Hemmung des bakteriellen Wachstums, die bei sub-MHC-Konzentrationen von 7-O-Malonylmacrolactin A beobachtet wurde zeigt, dass diese Verbindung auch in sehr geringer Konzentration zumindest gegen einige der getesteten Stämme bakteriostatisch wirkt.

Auch ein Vergleich der antimikrobiellen Aktivität von 7-O-Malonylmacrolactin A mit denen von Vergleichsverbindungen in Flüssigkultur zeigt, dass die minimal erforderlichen bakteriostatischen Konzentrationen (MBC), bei welcher eine überwiegende Hemmung des bakteriellen Wachstums beobachtet wurde, für 7-O-Malonylmacrolactin A sehr niedrig lagen.

**Tabelle 4: Antimikrobielle Aktivität von 7-O-Malonylmacrolactin A und Vergleichsverbindungen gegenüber klinischen Isolaten und Referenzstämmen**

| Stamm | 7-O-Malonyl-macrolactin A | | Vancomycin | Ampicillin | Erythromycin | Gentamycin | MCZ |
|---|---|---|---|---|---|---|---|
| | MHC | MBC | MHC | | | | |
| *S. aureus ***, # | >128 | 1 | 0,125 | 0,06 | 0,06 | 0,25 | NB |
| MRSA 2 | >128 | 4 | 2 | >128 | >128 | 128 | NB |
| MRSA 3 | >128 | 4 | 1 | 64 | >128 | 128 | NB |
| *E. faecalis* ATCC 29212 | 128 | 4 | 2 | 0,5 | 2 | 32 | NB |
| *E. faecalis* VRAS E305 | >128 | 0,06 | >128 | 0,5 | 128 | 64 | NB |
| *E. faecium* VRAR E315 | >128 | 4 | >128 | >128 | 2 | 64 | NB |
| *B. cepacia* WT139 | >128 | - | NB | >128 | >128 | >128 | NB |
| *B. cepacia* SCV 141 | 128 | 32 | NB | 128 | 128 | >128 | NB |
| *Candida parapsilosis* DSM5784 | 128 | - | NB | NB | NB | NB | 2 |
| *Candida krusei* DSM 6128 | >128 | 32 | NB | NB | NB | NB | 2 |
| *Candida albicans* DSM 11225 | >128 | - | NB | NB | NB | NB | 2 |

| | | | | | | | |
|---|---|---|---|---|---|---|---|
| Konzentrationen sind in µg/mL. NB = Nicht bestimmt, MHC = minimale Hemmkonzentration, MBC = minimale bakteriostatische bzw. fungistatische Konzentration, MCZ = Miconazol, ein Fungizid. | | | | | | | |

Die in Tabelle 4 dargestellten Ergebnisse zeigen, dass 7-O-Malonylmacrolactin A im Vergleich zu bekannten Antibiotika bei geringen Konzentrationen in derselben Größenordnung aktiv ist. Dabei ist es für 7-O-Malonylmacrolactin A nicht erforderlich, die MHC zur erreichen, sondern es reicht für die starke bakeriostatische Wirkung aus, dass die minimale bakteriostatische Konzentration erreicht wird. Weiterhin zeigt 7-O-Malonylmacrolactin A am Beispiel von *Candida* eine Wirkung gegen pathogene Hefen.

### Beispiel 6: Biozide Wirkung von 7-O-Malonylmacrolactin A gegenüber pro- und eukaryotischen Mikroorganismen

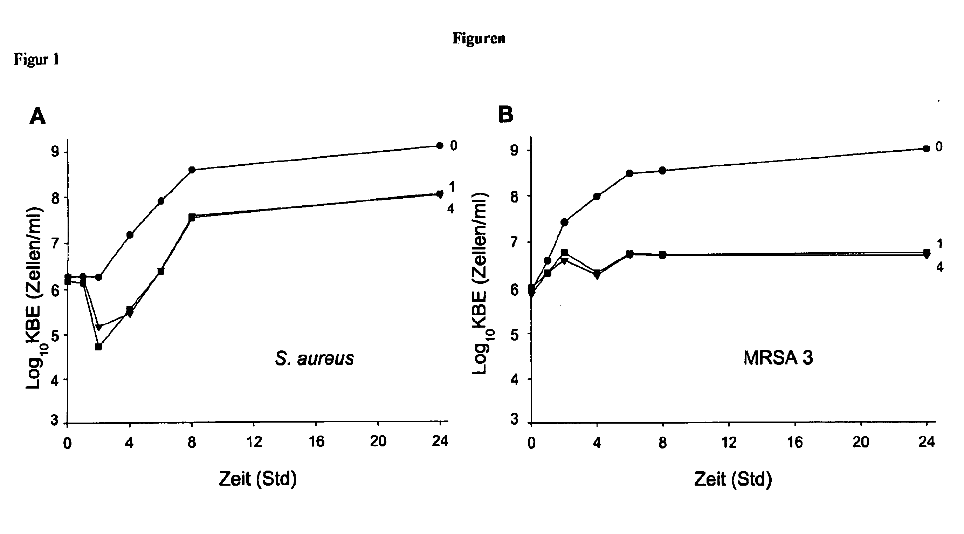

Zur Untersuchung der bakteriostatischen Wirkung von 7-O-Malonylmacrolactin A wurde die Wirkung von Konzentrationen unterhalb der MHC getestet. Die Wachstumskinetik von *Staphylococcus aureus* (#), in Figur 1A dargestellt, zeigt, dass 7-O-Malonyl-macrolactin A dieses Bakterium innerhalb der ersten 4 Stunden der Behandlung auch bei Konzentrationen deutlich unterhalb der MHC abtötet. Die anschließende Kultivierungszeit zeigt, dass das Wachstum dieser Bakterien um den Faktor 10 vermindert wird. Die Wirkung von 7-O-Malonylmacrolactin A gegenüber MRSA 3 (Figur 1 B) zeigt sich erst nach längerer Kultivierungszeit, jedoch wird das Wachstum dieses Bakterienstammes nach 4 Stunden fast vollständig gehemmt. 24 Stunden nach der Behandlung waren die Titer lebensfähiger Zellen um mehr als den Faktor 100 niedriger als bei der Kontrolle ohne 7-O-Malonylmacrolactin A.

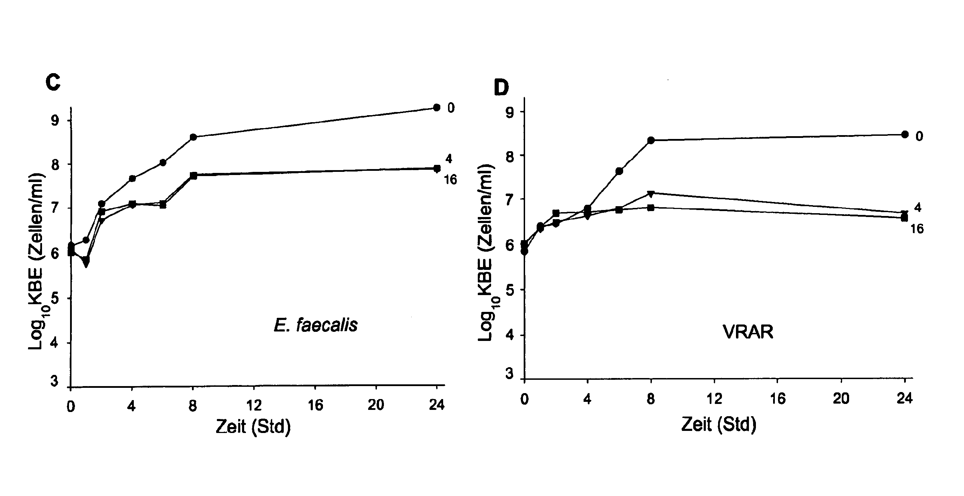

Nach 4 Stunden Kultivierung hemmte 7-O-Malonylmacrolactin A das Wachstum von *Enterococcus faecalis* ATCC 29212, wobei die Titer lebensfähiger Zellen um den Faktor 10 unterhalb der unbehandelten Kontrollkultur während der letzten Phase der Kultivierungszeit blieben (Figur 1C).

Vier Stunden nach Kultivierung mit 7-O-Malonylmacrolactin A wurde das Wachstum von *Enterococcus faecium* E 315, Vancomycin-resistent, Ampicillin-resistent, fast vollständig gehemmt (Figur 1D).

Interessanterweise zeigt 7-O-Malonylmacrolactin A eine stärkere bakteriostatische Wirkung gegenüber Antibiotika-resistenten Bakterien, die aus klinischen Patienten isoliert wurden, als gegenüber nicht-resistenten Stämmen. Dabei erhöht sich die Lebendzellzahl während der Kultivierung mit 7-O-Malonylmacrolactin A nicht wesentlich. Insbesondere der Vergleich von Figur 1A mit Figur 1B macht deutlich, dass die bakteriostatische Wirkung von 7-O-Malonylmacrolactin A gegen den Antibiotika-resistenten MRSA 3 gleich groß oder größer ist, als gegen den Referenzstamm *S. aureus* ^{#}*,* der keine Antibiotika-Resistenzen trägt.

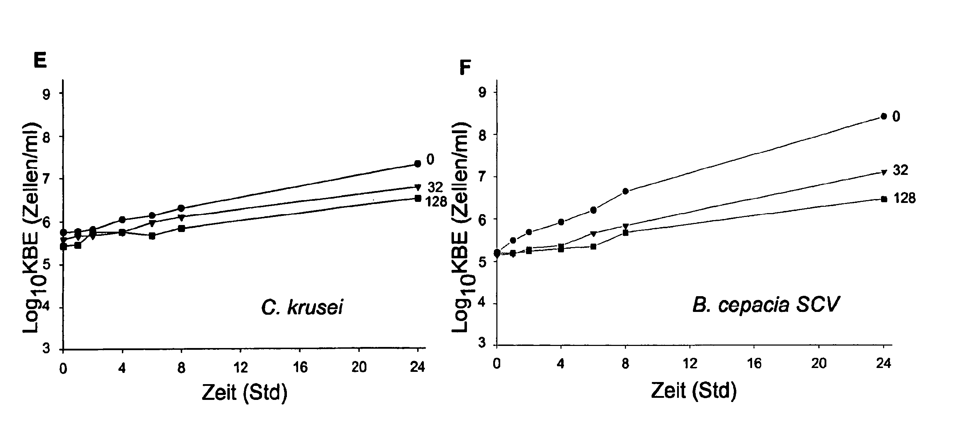

Auch gegenüber dem eukaryotischen Mikroorganismus *Candida krusei* (Figur 1E) sowie gegenüber dem von sich aus gegenüber bekannten Antibiotika resistentem *Burkholderia cepacia* SCV 141 (Figur 1F) zeigt 7-O-Malonylmacrolactin A antbiotische Wirkung und hemmt das Wachstum jeweils um bis zu einem Faktor von 10 bei Konzentrationen von 128 µg/mL (*C. krusei*) bzw. 32 µg/mL (*B. cepacia*).

Bei der Untersuchung der Hemmwirkung von 7-O-Malonylmacrolactin A wurde generell keine Dosisabhängigkeit festgestellt, wenn in Experimenten zwei Konzentrationen bei sub-MHCs eingesetzt wurden.

### Beispiel 7: Störung der bakteriellen Zellteilung durch 7-O-Malonylmacrolactin A

Die Untersuchung von Bakterienstämmen aus klinischen Isolaten, die gemäß Beispiel 5 in Schüttelkulturen bereits durch Konzentrationen von 7-O-Malonylmacrolactin A unterhalb der MHC deutlich in ihrem Wachstum gehemmt werden zeigt, dass 7-O-Malonylmacrolactin A den Ablauf der Zellteilung stört. Es wird vermutet, dass 7-O-Malonylmacrolactin A die Teilung von Bakterienzellen hemmt, so dass deren Vermehrung unterbrochen wird. Dabei ist 7-O-Malonylmacrolactin A wirksamer als das bekannte 7-O-Succinylmacrolactin A oder Macrolactin A.

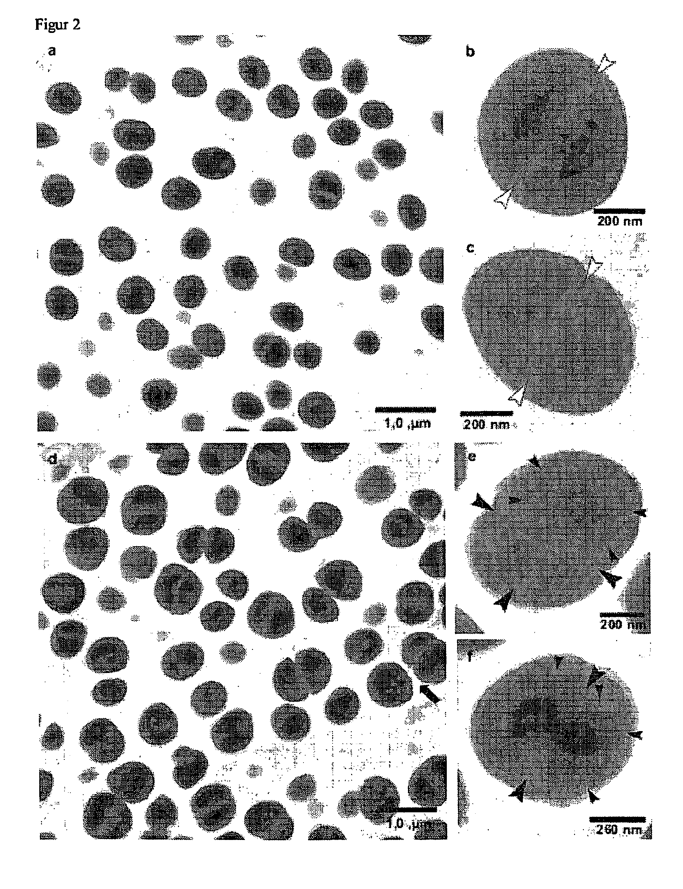

Die antibiotische Wirkung von 7-O-Malonylmacrolactin A tritt bereits bei Konzentrationen deutlich unterhalb der MHC auf, so dass zur medizinischen Anwendung auch Dosen eingesetzt werden können, die unterhalb der MHC eine wirksame Konzentrationen darstellen. Insbesondere in Kombination mit der geringen zytotoxischen Wirkung von 7-O-Malonylmacrolactin A, vor allem bei geringen Konzentrationen, ergibt sich daraus der Vorteil, bereits in geringer Dosierung eine antibiotische Wirkung zu erreichen. Elektronenmikroskopische Aufnahmen des klinischen Isolats von *Staphylcoccus aureus* (MRSA 3, Methicillin resistent), in Figur 2 dargestellt, zeigen die Wirkung von 7-O-Malonylmacrolactin A. Die Figuren 2a) bis c) zeigen unbehandelte Schüttelkulturen nach 4 h Inkubation, Figuren 2d) bis f) gleiche Kulturen, jedoch mit einem Zusatz von 16 µg/mL 7-O-Malonylmacrolactin A. Diese Konzentration liegt deutlich unterhalb der MHC, hemmt jedoch das Wachstum deutlich, wie auch aus Figur 1B für Konzentrationen von 1 und 4 µg/mL erkennbar ist. Das 7-O-Malonylmacrolactin A beeinflußt die Zellteilung des MRSA 3, wie anhand der veränderten Teilungsebenen zu sehen ist. Die unbehandelten Zellen in Figuren 2a) bis c) lassen die Teilungsebene als helle Querwand erkennen, die mit weißen Pfeilspitzen (Figuren 2b) und c)) gekennzeichnet sind. Die behandelten MRSA 3 zeigen jedoch eine gestörte Zellteilung. Die großen schwarzen Pfeilspitzen (Figuren 2e) und f)) zeigen die Teilungsebene, die kleinen schwarzen Pfeilspitzen deuten eine asymmetrische Initiierung der Zellteilung an.

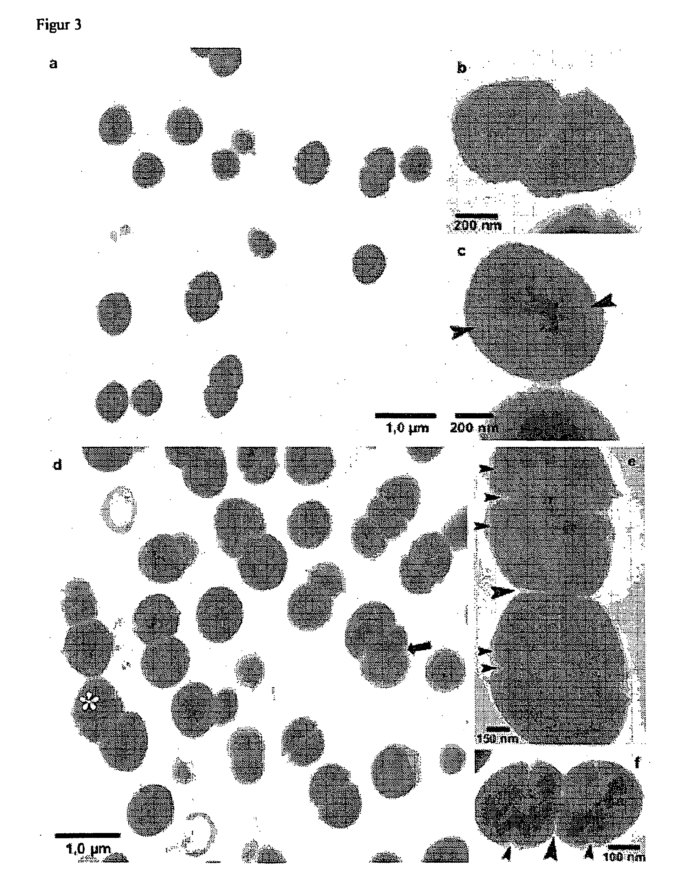

Die Wirkung von 7-O-Malonylmacrolactin A auf die Zellteilung wird auch in den elektronenmikroskopischen Aufnahmen von *Enterococcus faecium* (VRAR = Vancomycin resistent, Ampicillin resistent, E315, klinisches Isolat) deutlich, die in Figur 3 wiedergegeben sind. Die Zellteilungsebenen unbehandelter Zellen nach 4 Stunden Wachstum ohne Antibiotikum sind in Figuren 3a), b) und c) hell sichtbar. In Figuren 3d) bis f), den Ansätzen mit 16 µg/mL 7-O-Malonylmacrolactin A, sind keine vollständig ausgebildeten Zellteilungsebenen erkennbar, sondern es wird eine anomale, asymmetrische Zellteilung initiiert (große schwarze Pfeilspitzen). Dabei ist jedoch keine ausgebildete Zellteilungsebene zu erkennen. Die Initiierung der asymmetrischen Zellteilung wird durch kleine schwarze Pfeilspitzen markiert. Als ein Ergebnis der gestörten Zellteilung ist eine pseudomultizelluläre Kette, mit einem weißen Stern markiert (Figur 3d), sichtbar (Figur 3f), die ebenfalls keine ausgebildeten Septen in der Ebene aufweist, in der bei unbehandelten Zellen (Figuren 3a) und b)) die Zellteilung optisch hell erkennbar ist.

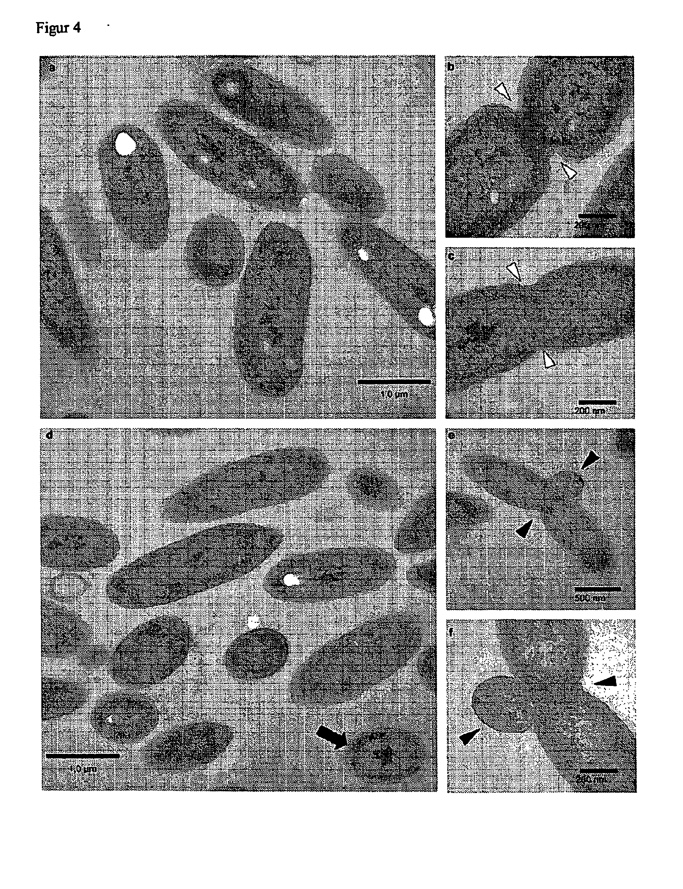

Weiterhin belegen elektronenmikroskopische Aufnahmen die bakteriostatische Wirkung von 7-O-Malonylmacrolactin A gegenüber Gram-negativen Bakerien am Beispiel der Zellteilung von *Burkholderia cepacia.* So zeigen die Figuren 4a, b und c *Burkholderia cepacia* SCV (klinisches Isolat) in Schüttelkultur ohne Zusatz antibiotischer Wirkstoffe (Kontrolle) und Figuren 4d, e und f nach 4 h Wachstum in Schüttelkultur in Gegenwart von 128 µg/mL 7-O-Malonylmacrolactin A. Weiße Pfeilspitzen weisen bei den Kontrollen auf die Zellteilungsebene und die eingezogene Querwand hin. Bei den mit 7-O-Malonylmacrolactin A behandelten Gram-negativen Zellen (Figuren 4e und f) wird mit schwarzen Pfeilspitzen auf die Zellteilungsebenen und unnatürlichen Aussprossungen hingewiesen. In den mit 7-O-Malonylmacrolactin A behandelten Zellen wurden auch Zellen anomaler Größen beobachtet, wie in Figur 4d mit einem schwarzen Pfeil angedeutet.

### Beispiel 8: Wirkung von 7-O-Malonylmacrolactin A gegenüber Mikroorganismen, insbesondere gegenüber Gram-negativen Bakterien und Eukaryonten

Als ein Beispiel für die Wirkung gegenüber eukaryotischen Mikroorganismen am Beispiel von Hefen wurde 7-O-Malonylmacrolactin A gegenüber *Candida krusei* DSMZ 6128 getestet. Das Resultat ist in Figur 2e dargestellt und zeigt eine gewisse Hemmwirkung. Auch gegenüber dem Gram-negativen Bakterium *Burkholderia cepacia* SCV 141 zeigt 7-O-Malonylmacrolactin A eine Hemmwirkung (Figur 2f).

### Beispiel 9: Zytotoxische Wirkung von 7-O-Malonylmacrolactin A, 7-O-Succinylmacrolactin A und Macrolactin A gegenüber tierischen Zellen

Die Zytotoxizität von 7-O-Malonylmacrolactin A wurde im Vergleich mit 7-O-Succinylmacrolactin A und Macrolactin A *in vitro* anhand der Inhibierung der Proliferation von L929 Maus-Fibroblastenzellen bzw. der menschlichen Epithelzelllinie HeLa getestet.

Für die Zellkultur wurde die HeLa-Zellen in DMEM-Medium (Gibco) mit geringem Glucosegehalt, die L 929 Zellen in DMEM-Medium mit hohem Glucosegehalt, jeweils supplementiert mit 10 Vol.-% fötalem Kälberserum (Gibco) bei 37 °C in einer Atmosphäre mit 5% CO₂ kultiviert. Die Zellen wurden aus Stammkulturen durch Trypsinieren mit EDTA (HeLa) bzw. ohne EDTA (L 929) gewonnen, gezählt, und auf 2 x 10⁵ Zellen/mL verdünnt. Für die Tests wurden Mikrotiterplatten (Nunc) mit 96 Näpfen ohne bzw. in Gegenwart der Testverbindungen oder Methanol in Reihenverdünnung ausplattiert. Morphologische Veränderungen der Zellen wurden durch Phasenkontrast-Mikroskopie nach 1, 2 und 5 Tagen Inkubierung bestimmt.

Nach 5-tägiger Kultivierung wurden die Zellzahlen unter Verwendung des Zellproliferationstests CyQUANT (Molecular Probes) bestimmt, einem sehr empfindlichen Mikrotiterplattentest auf Basis von Fluoreszenz. Der Test verwendet den Farbstoff CyQUANT, der eine hohe Verstärkung der Fluoreszenz beim Binden an zelluläre Nukleinsäuren erzeugt, die durch Anregung unter Verwendung von Fluoreszein gemessen werden kann. Die Emission der Fluoreszenz des Komplexes Farbstoff - Nukleinsäure korrelierte linear mit der Zellzahl. Für den Test wurde der Überstand nach 5-tägiger Inkubation vorsichtig entfernt, die Zellen wurden mit PBS (Phosphat gepufferte Salzlösung) gewaschen, der Puffer wurde entfernt und die Zellen bei -80 °C eingefroren. Zur Zeit des Tests wurden die Zellen bei Raumtemperatur aufgetaut und in Puffer lysiert, der den Farbstoff CyQUANT enthielt, gemäß den Anweisungen des Herstellers. Die Fluoreszenz wurde mit einem fluorometrischen Mikrotiterplattenleser (Titertex Fluoroskan II) gemessen (Anregung bei 480 nm, Emission bei 520 nm). Die Absorbanzwerte wurden verwendet, um den Prozentanteil der Proliferation von Zellen in Medium allein und in Gegenwart der Reihenverdünnungen von Methanol und den Macrolactin-Verbindungen zu berechnen.

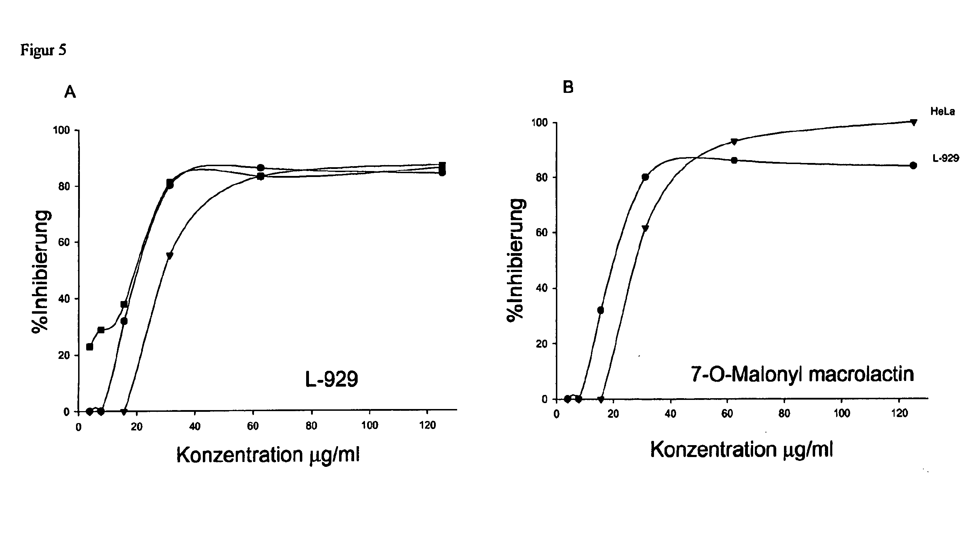

Die Zellen wurden in DMEM-Medium nach Erreichen einer Zellzahl von ca. 10⁴ - 2 x 10⁵/mL einem Austausch des Mediums gegen frisches mit den in Figuren 5A bzw. 4B von 7-O-Malonylmacrolactin A bzw. in Tabelle 5 verschiedener Macrolactine angegebenen Konzentrationen unterzogen. Die Proliferation wurde mit dem Fluoreszenz-basierten Test CyQUANT zur Bestimmung der Lebendzellzahl gemessen. Die relative Inhibierung der Proliferation gegenüber einer Vergleichskultur mit frischem Medium ohne Zusatz eines Macrolactins sind in Tabelle 5 gezeigt.

**Tabelle 5: Inhibierung der Proliferation durch Macrolactine**

| | Konzentration der Verbindung [µg/mL] | | | | | | |
|---|---|---|---|---|---|---|---|
| Verbindung | Zellen | 125 | 56,5 | 31,25 | 15,6 | 7,8 | 3,9 |
| 7-O-Malonylmacrolactin A | HeLa | 100% | 93% | 62% | 0 | 0 | 0 |
| 7-O-Malonylmacrolactin A | L929 | 84% | 86% | 80% | 32% | 0 | 0 |
| 7-O-Succinylmacrolactin A | L929 | 87% | 83% | 55% | 0 | 0 | 0 |
| 7-0-Macrolactin A | L929 | 83% | 83% | 81% | 38% | 29% | 23% |

In Figur 5A wird deutlich, dass Macrolactin A (■) die Proliferation der Mauszellen L929 ebenso wie 7-O-Succinylmacrolactin A (▼) stärker hemmt, als 7-O-Malonylmacrolactin A (•).

Die Figur 5B zeigt, dass die Proliferation menschlicher Zellen, hier am Beispiel von HeLa-Zellen (V), in geringerem Maße als diejenige von Mausfibroblastenzellen L-929 (•) durch 7-O-Malonylmacrolactin A gehemmt wird. Insbesondere bei niedrigen Konzentrationen ist die Hemmung der Proliferation menschlicher Zellen geringer als die von Mauszellen.

Das 7-O-Malonylmacrolactin A war für sowohl den die getesteten menschlichen Epithelzellen als auch die Fibroblastenzellen zytotoxisch, dies jedoch nur bei höheren Konzentrationen.

Für Macrolactin A ist bereits gezeigt worden, dass es zusätzlich zu antibakteriellen Wirkungen zytotoxische und antivirale Wirkung hat. Das vorliegend isolierte 7-O-Malonylmacrolactin A zeigt bei höheren Konzentrationen eine höhere Zytotoxizität als die getesteten Vergleichsverbindungen.

7-O-Malonylmacrolactin A hemmte die Proliferation von HeLa-Zellen bei einer Konzentration von 15,6 µg/mL nicht, hingegen wurde die Fibroblastenzelllinie bei dieser Konzentration zu ca. 32% gehemmt. Insgesamt zeigt 7-O-Malonyl-Macrolactin A gegenüber den getesteten Fibroblastenzellen eine stärkere Inhibierung als 7-O-Succinyl-Macrolactin A, wobei bei einer Konzentration von 7,8 µg/mL gleichfalls keine Inhibierung mehr beobachtet wurde. Im Vergleich mit Macrolactin A zeigt 7-O-Malonylmacrolactin A gegenüber den getesteten Fibroblastenzellen bei hohen Konzentrationen bis zur getesteten Konzentration von 15,6 µg/mL eine ähnliche bzw. leicht geringere Inhibierung der Proliferation, bei den Konzentrationen von 7,8 und 3,9 µg/mL jedoch im Unterschied zu Macrolactin A keine Hemmung der Proliferation mehr.

Da die Konzentrationen, ab der die zytotoxische Wirkung auftritt, für 7-O-Malonylmacrolactin A oberhalb der Konzentration liegt, bei der insbesondere bei antibiotikaresistenten Bakterien eine starke bakteriostatische Wirkung auftritt, kann 7-O-Malonyl-Macrolactin A zur Herstellung pharmazeutischer Präparate zur medizinischen Anwendung eingesetzt werden. Ein besonderer Vorteil des 7-O-Malonylmacrolactins A gegenüber dem 7-O-Succinylmacrolactin A bzw. Macrolactin A ist dessen höhere antimikrobielle Aktivität, insbesondere bei sehr niedrigen Konzentrationen, und die geringere Zytotoxizität gegenüber menschlichen Zellen, insbesondere im Vergleich mit Macrolactin A.

Bei der mikroskopischen Untersuchung der Wirkung von 7-O-Malonylmacrolactin auf die menschlichen Epithelzellen HeLa und die Maus-Fibroblastenzellen L 929 wurde gefunden, dass die menschliche Zelllinie in geringerem Maße als die der Maus durch 7-O-Malonylmacrolactin A beeinträchtigt wird. Die anti-proliferative Wirkung beruht auf einem toxischen Effekt, da die Morphologie der behandelten Zellen rund war. Die zur Kontrolle mit Methanol behandelten Zellen wurden in ihrer Proliferation nicht beeinträchtigt.

Die Quantifizierung des zytotoxischen Effekts von 7-O-Malonylmacrolactin A unter Verwendung des CyQUANT-Tests zeigte, dass die Verbindung in der Lage war, die Proliferation von HeLa-Zellen bei 31,25 µg/mL zu hemmen. Die mit 62,5 µg/mL 7-O-Malonylmacrolactin A behandelten Zellen zeigten eine fast vollständige Hemmung des Wachstums.

## Patentansprüche

1. 7-O-Malonylmacrolactin A.

2. Verwendung von 7-O-Malonylmacrolactin A zur Herstellung von Zusammensetzungen zur medizinischen Verwendung.

3. Verwendung von 7-O-Malonylmacrolactin A zur Herstellung pharmazeutischer Zusammensetzungen zur medizinischen Verwendung gegen Infektionen.

4. Verwendung nach Anspruch 3, **gekennzeichnet durch** die Kombination von 7-O-Malonylmacrolactin A mit 7-O-Succinylmacrolactin A.

5. Verwendung nach Anspruch 3 oder 4, **dadurch gekennzeichnet, dass** die Infektion bakteriell ist, wobei das infizierende Bakterium zumindest eine Resistenz gegen andere antibiotische Verbindungen aufweist.

6. Verwendung nach Anspruch 5, **dadurch gekennzeichnet, dass** die andere antibiotische Verbindung aus der Gruppe ausgewählt ist, die Vancomycin, Methicillin, Ampicillin und Erythromycin umfasst.

7. Pharmazeutische Zusammensetzung enthaltend 7-O-Malonylmacrolactin A nach Anspruch 1, **gekennzeichnet durch** eine Verwendung nach einem der Ansprüche 2 bis 6.

8. Pharmazeutische Zusammensetzung nach Anspruch 7, **dadurch gekennzeichnet, dass** sie geeignet ist, eine wirksame Konzentration im Bereich von 0,01 bis 50 µg/mL einzustellen.

9. Verfahren zur Herstellung von 7-O-Malonylmacrolactin A, **gekennzeichnet durch** die Verwendung des Stammes DSM 16696.

10. Verwendung von *Bacillus subtilis* der Hinterlegungsnummer DSM 16696 in einem Verfahren nach Anspruch 9.

11. 7-O-Malonylmacrolactin A nach Anspruch 1 zur Verwendung als pharmazeutischer Wirkstoff.

12. 7-O-Malonylmacrolactin A nach Anspruch 11, **dadurch gekennzeichnet, dass** der pharmazeutische Wirkstoff zur medizinischen Verwendung gegen Infektionen ist.

## Claims

1. 7-O-Malonylmacrolactine A.

2. Use of 7-O-Malonylmacrolactine A for the production of compositions for medical use.

3. Use of 7-O-Malonylmacrolactine A for the production of pharmaceutical compositions for medical use against infections.

4. Use according to claim 3, **characterized by** the combination of 7-O-Malonylmacrolactine with 7-O-Succinylmacrolactine A.

5. Use according to claim 3 or 4, **characterized in that** the infection is bacterial, wherein the infecting bacterium has at least one resistance against other antibiotic compounds.

6. Use according to claim 5, **characterized in that** the other antibiotic compound is selected from the group comprising vancomycin, methicillin, ampicillin and erythromycin.

7. Pharmaceutical composition comprising 7-O-Malonylmacrolactine A according to claim 1, **characterized by** a use according to one of claims 2 to 6.

8. Pharmaceutical composition according to claim 7, **characterized in that** it is suitable for adjusting an effective concentration in a range from 0.01 to 50 µg/mL.

9. Process for the production of 7-O-Malonylmacrolactine A, **characterized by** the use of the strain DSM and 16696.

10. Use of *Bacillus subtilis* of the accession number DSM and 16696 in a process according to claim 9.

11. 7-O-Malonylmacrolactine A according to claim 1 for use as a pharmaceutically active compound.

12. 7-O-Malonylacrolactine A according to claim 11, **characterized in that** the pharmaceutically active compound is for medical use against infections.

## Revendications

1. 7-O-Malonylmacrolactine A.

2. Utilisation de la 7-O-Malonylmacrolactine A pour la fabrication de compositions destinées à l'usage médical.

3. Utilisation de la 7-O-Malonylmacrolactine A pour la fabrication de compositions pharmaceutiques destinées à l'usage médical pour lutter contre les infections.

4. Utilisation selon la revendication 3, **caractérisée par** la combinaison de la 7-O-Malonylmacrolactine A avec la 7-O-Succinylmacrolactine A.

5. Utilisation selon la revendication 3 ou 4, **caractérisée par le fait que** l'infection est bactérienne, la bactérie infectante présentant au moins une résistance contre d'autres composés antibiotiques.

6. Utilisation selon la revendication 5, **caractérisée par le fait que** l'autre composé antibiotique est sélectionné dans le groupe comportant la vancomycine, la méthicilline, l'ampicilline et l'erythromycine.

7. Composé pharmaceutique comprenant de la 7-O-Malonylamacrolactine A selon la revendication 1, **caractérisé par** une utilisation selon une des revendications 2 à 6.

8. Composé pharmaceutique selon la revendication 7, **caractérisé par le fait qu'**il est adapté pour ajuster une concentration efficace dans la plage située entre 0,01 et 50 µg/mL.

9. Procédé destiné à la fabrication de la 7-O-Malonylmacrolactine A, **caractérisé par** l'utilisation de la souche DSM 16696.

10. Utilisation de *Bacillus subtilis* ayant le numéro d'ordre de dépôt DSM 16696 dans un procédé selon la revendication 9.

11. 7-O-Malonylmacrolactine A selon la revendication 1 pour utilisation comme substance active pharmaceutique.

12. 7-O-Malonylmacrolactine A selon la revendication 11, **caractérisé par le fait que** la substance active pharmaceutique est destinée à l'usage médical contre les infections.
